# EUROPÄISCHE PATENTSCHRIFT

(11) **EP 0 901 472 B1**
(45) Veröffentlichungstag und Bekanntmachung des Hinweises auf die Patenterteilung: **09.04.2003**
(21) Anmeldenummer: 97923034.9
(22) Anmeldetag: 09.05.1997
(51) Int. Cl.: C07D 233/84, A01N 43/50

(54) **MERCAPTO-IMIDAZOLYL-DERIVATE UND IHRE VERWENDUNG ALS MIKROBIZIDE**
MERCAPTO-IMIDAZOLYL DERIVATIVES AND THEIR USE AS MICROBICIDES
DERIVES DE MERCAPTO-IMIDAZOLYLE ET LEUR UTILISATION COMME MICROBICIDES

(30) Priorität: 21.05.1996 DE 19620408
(43) Veröffentlichungstag der Anmeldung: 17.03.1999
(73) Patentinhaber: Bayer CropScience AG, 40789 Monheim (DE)
(72) Erfinder: JAUTELAT, Manfred, D-51399 Burscheid (DE); TIEMANN, Ralf, D-51375 Leverkusen (DE); DUTZMANN, Stefan, D-40764 Langenfeld (DE); STENZEL, Klaus, D-40595 Düsseldorf (DE)
(86) Internationale Anmeldenummer: EP9702374
(87) Internationale Veröffentlichungsnummer: WO97044323

(56) Entgegenhaltungen:
- EP-A- 0 052 424
- EP-A- 0 251 086
- EP-A- 0 297 345
- DE-A- 2 324 010
- DE-A- 19 528 046
- CHEMICAL ABSTRACTS, vol. 89, no. 13, 25.September 1978 Columbus, Ohio, US; abstract no. 109238x, M.V. POVSTYANOI ET AL.: "Synthesis and biological activity of 2-mercaptoimidazole derivatives." Seite 897; Spalte 2; XP002039192 & KHIM.-FARM. ZH., Bd. 12, Nr. 5, 1978, Seiten 59-62,

## Beschreibung

Die vorliegende Erfindung betrifft neue Mercapto-imidazolyl-Derivate, mehrere Verfahren zu deren Herstellung und deren Verwendung als Mikrobizide.

Es ist bereits bekannt geworden, daß zahlreiche Imidazolyl-Derivate fungizide Eigenschaften besitzen (vgl. EP-A 0 015 756, EP-A 0 040 345, EP-A 0 052 424, EP-A 0 061 835, EP-A 0 297 345, EP-A 0 094 564, EP-A 0 196 038, EP-A 0 267 778, EP-A 0 378 953, EP-A 0 044 605, EP-A 0 069 442, EP-A 0 055 833, EP-A 0 301 393, DE-A 2 324 010, DE-A 2 737 489, DE-A 2 551 560, EP-A 0 065 485, DE-A 2 735 872, EP-A 0 234 242, DE-A 2 201 063, EP-A 0 145 294 und DE-A 3 721 786). Die Wirksamkeit dieser Stoffe ist gut, läßt aber bei niedrigen Aufwandmengen in manchen Fällen zu wünschen übrig.

Im einzelnen geht aus der EP - A 0 297 345 und der EP - A 0 052 424 hervor, daß bestimmte Triazole und Imidazole als Fungizide eingesetzt werden können. Die aufgeführten Verbindungen enthalten jedoch keine Mercapto - Gruppe am Azol - Ring.

Die EP - A 0 251 086 betrifft schließlich fungizid wirksame Triazol - Derivate, in denen der Heterocyclus durch einen Mercapto - Rest substituiert ist. Entsprechende Imidazol - Derivate werden aber nicht erwähnt.

Es wurden nun neue Mercapto-imidazolyl-Derivate der Formel 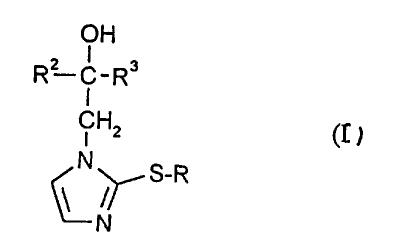 in welcher
- R: für Wasserstoff, Methyl, Ethyl, n - Propyl oder Isopropyl steht,
- R²: für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
- R³: für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl.
gefunden.

Zahlreiche der erfindungsgemäßen Stoffe enthalten eines oder mehrere asymmetrisch substituierte Kohlenstoffatome. Sie können daher in optischen Isomerenformen anfallen. Die vorliegende Erfindung betrifft sowohl die einzelnen Isomeren als auch deren Gemische.

Weiterhin wurde gefunden, daß man Mercapto - imidazolyl - Derivate der Formel (I) erhält, wenn man Imidazole der Formel 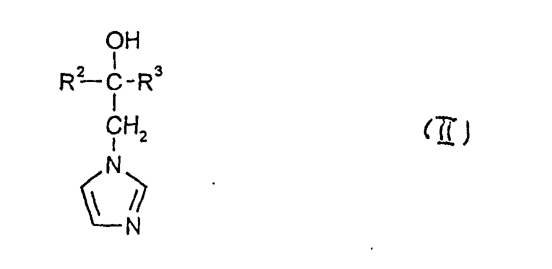 in welcher
R² und R³ die oben angegebenen Bedeutungen haben, entweder
a) nacheinander mit starken Basen und Schwefel in Gegenwart eines Verdünnungsmittels umsetzt und dann mit Wasser, gegebenenfalls in Gegenwart einer Säure hydrolysiert,
   oder
b) mit Schwefel in Gegenwart eines hoch siedenden Verdünnungsmittels umsetzt und dann gegebenenfalls mit Wasser sowie gegebenenfalls mit Säure behandelt,
und gegebenenfalls die nach den Varianten (a) und (b) entstehenden Verbindungen der Formel 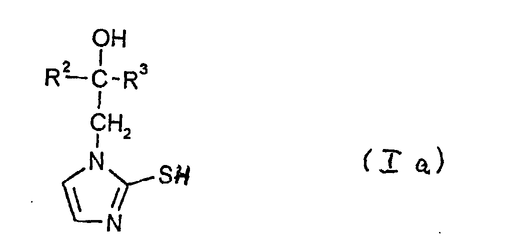 in welcher
R² und R ³ die oben angegebenen Bedeutungen haben,
mit Halogen-Verbindungen der Formel

R⁴-Hal (III)

in welcher
- R⁴: für Methyl, Ethyl, n- Propyl oder Isopropyl steht und
- Hal: für Chlor, Brom oder Iod steht,
in Gegenwart eines Säurebindemittels und in Gegenwart eines Verdünnungsmittels umsetzt.

Schließlich wurde gefunden, daß die neuen Mercapto - imidazolyl - Derivate der Formel (I) sehr gute
mikrobizide Eigenschaften aufweisen und sowohl im Pflanzenschutz als auch im Materialschutz eingesetzt werden können.

Überraschenderweise besitzen die erfindungsgemäßen Stoffe eine bessere mikrobizide Wirksamkeit als die konstitutionell ähnlichsten, vorbekannten Verbindungen gleicher Wirkungsrichtung.

Die erfindungsgemäßen Mercapto-imidazolyl-Derivate der Formel (I), in denen R für Wasserstoff steht, können in der "Mercapto"-Form der Formel 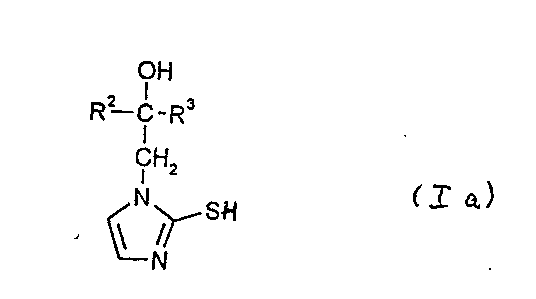 oder in der tautomeren "Thiono"-Form der Formel 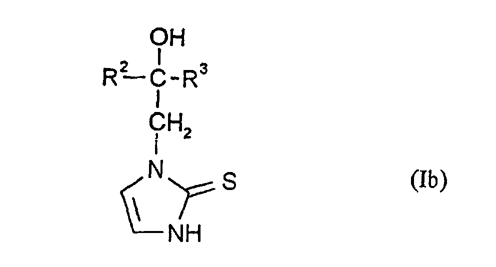 vorliegen. Der Einfachheit halber wird jeweils nur die "Mercapto"-Form aufgeführt.

Als Beispiele für erfindungsgemäße Stoffe seien die in den folgenden Tabellen aufgeführten Mercapto-imidazolyl-Derivate genannt.

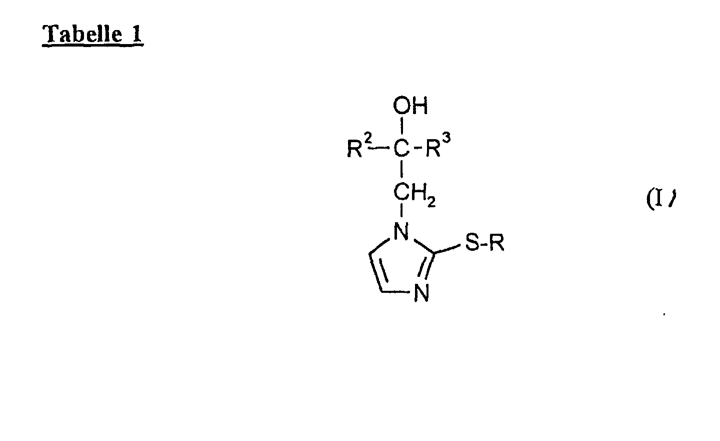

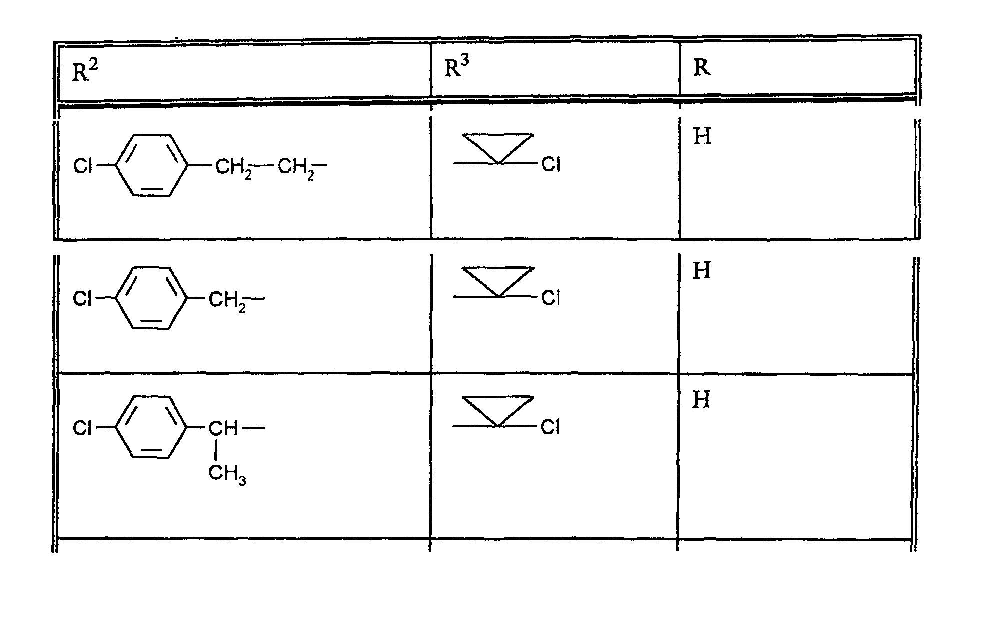

Verwendet man 2-(1-Chlor-cyclopropyl)-1-(2-chlor-phenyl)-3-(imidazol-1-yl)-propan-2-ol als Ausgangsstoff, n-Butyl-lithium als starke Base und Schwefel-Pulver als Reaktionskomponente, so kann der Verlauf der ersten Stufe des erfindungsgemäßen Verfahrens (Variante a) durch das folgende Formelschema veranschaulicht werden:

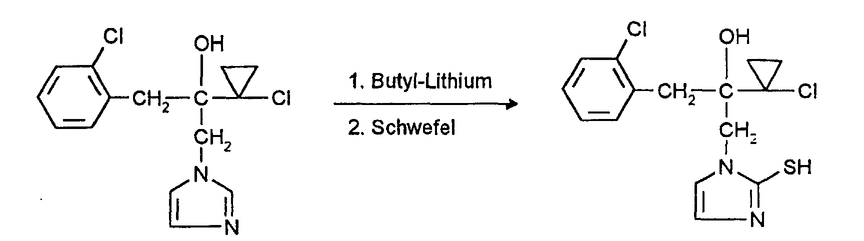

Verwendet man 2-(1-Chlor-cyclopropyl)-1-(2-chlor-phenyl)-3-(imidazol- 1 -yl)-propan-2-ol als Ausgangsstoff, Schwefel-Pulver als Reaktionskomponente und N-Methyl-pyrrolidon als Verdünnungsmittel, so kann der Verlauf der ersten Stufe des erfindungsgemäßen Verfahrens (Variante b) durch das folgende Formelschema veranschaulicht werden:

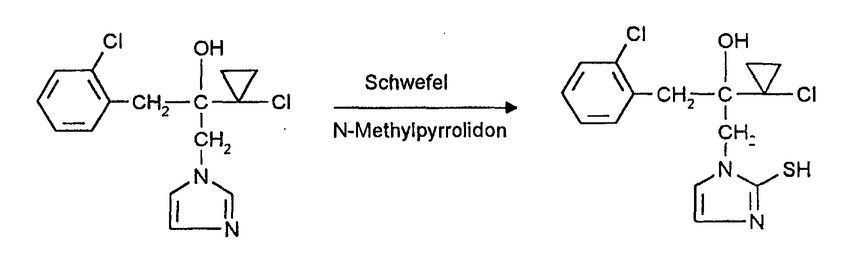

Verwendet man 2-(1-Chlor-cyclopropyl)-1-(2-chlorphenyl)-3-(2-mercapto-imidazol-1-yl)-propan-2-ol als Ausgangsstoff und Methyliodid als Reaktionskomponente, so kann der Verlauf der zweiten Stufe des erfindungsgemäßen Verfahrens durch das folgende Formelschema veranschaulicht werden:

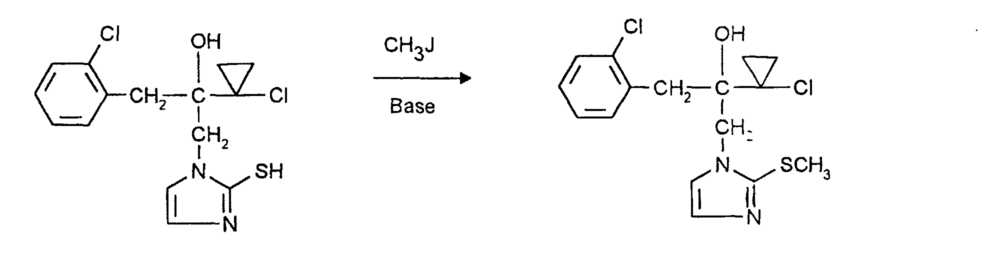

Die bei der Durchführung des erfindungsgemäßen Verfahrens als Ausgangsstoffe benötigten

Imidazole der Formel (II) sind bekannt oder lassen sich nach bekannten Methoden herstellen (vgl. EP-A 0 015 756, EP-A 0 040 345, EP-A 0 052 424, EP-A 0 061 835, EP-A 0 297 345, EP-A 0 094 564, EP-A 0 196 038, EP-A 0 267 778, EP-A 0 378 953, EP-A 0 044 605, EP-A 0 069 442, EP-A 0 055 833, EP-A 0 301 393, DE-A 2 324 010, DE-A 2 737 489, DE-A 2 551 560, EP-A 0 065 485, DE-A 2 735 872, EP-A 0 234 242, DE-A 2 201 063, EP-A 0 145 294 und DE-A 3 721 786).

Als Basen kommen bei der Durchführung der ersten Stufe des erfindungsgemäßen Verfahrens (Variante a), alle für derartige Reaktionen üblichen, starken Alkalimetall-Basen in Betracht. Vorzugsweise verwendbar sind n-Butyl-lithium, Lithiumdiisopropyl-amid, Natriumhydrid, Natriumamid und auch Kalium-tert.-butylat im Gemisch mit Tetramethylethylen-diamin (= TMEDA).

Bei der Durchführung der ersten Stufe des erfindungsgemäßen Verfahrens (Variante a), kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird vorzugsweise in Form von Pulver eingesetzt. Zur Hydrolyse verwendet man bei der Durchführung der ersten Stufe des erfindungsgemäßen Verfahrens (Variante a). Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung der ersten Stufe des erfindungsgemäßen Verfahrens (Variante a), innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung aller Schritte des erfindungsgemäßen Verfahrens arbeitet man im allgemeinen unter Normaldruck. Es ist aber auch möglich, unter erhöhtem oder vermindertem Druck zu arbeiten. So kommt vor allem bei der Durchführung der ersten Stufe des erfindungsgemäßen Verfahrens nach Variante (b) ein Arbeiten unter erhöhtem Druck in Frage.

Bei der Durchführung der ersten Stufe des erfindungsgemäßen Verfahrens nach Variante (a) setzt man auf I Mol an Imidazol der Formel (II) im allgemeinen 2 bis 3 Äquivalente, vorzugsweise 2,0 bis 2,5 Äquivalente, an starker Base und anschließend eine äquivalente Menge oder auch einen Überschuß an Schwefel ein. Die Umsetzung kann unter Schutzgas-atmosphäre, z.B. unter Stickstoff oder Argon, vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Bei der Durchführung der ersten Stufe des erfindungsgemäßen Verfahrens nach Variante (b) kommen als Verdünnungsmittel alle für derartige Umsetzungen üblichen, hoch siedenden organischen Solventien in Betracht. Vorzugsweise verwendbar sind Amide, wie Dimethylformamid und Dimethylacetamid, außerdem heterocyclische Verbindungen, wie N-Methyl-pyrrolidon, und auch Ether, wie Diphenylether.

Schwefel wird auch bei der Durchführung der ersten Stufe des erfindungsgemäßen Verfahrens nach der Variante (b) im allgemeinen in Form von Pulver eingesetzt. Nach der Umsetzung kann gegebenenfalls eine Behandlung mit Wasser sowie gegebenenfalls mit Säure vorgenommen werden. Diese wird so durchgeführt wie die Hydrolyse bei der Durchführung der ersten Stufe des erfindungsgemäßen Verfahrens nach der Variante (a).

Die Reaktionstemperaturen können auch bei der Durchführung der ersten Stufe des erfindungsgemäßen Verfahrens nach Variante (b) innerhalb eines größeren Bereiches variiert werden. Im allgemeinen arbeitet man bei Temperaturen zwischen 150°C und 300°C, vorzugsweise zwischen 180°C und 250°C.

Bei der Durchführung der ersten Stufe des erfindungsgemäßen Verfahrens nach Variante (b) setzt man auf 1 Mol an Imidazol der Formel (II) im allgemeinen 1 bis 5 Mol, vorzugsweise 1,5 bis 3 Mol an Schwefel ein. Die Aufarbeitung erfolgt nach üblichen Methoden. Im allgemeinen geht man so vor, daß man das Reaktionsgemisch mit einem in Wasser nur wenig löslichen organischen Solvens extrahiert, die vereinigten organischen Phasen trocknet und einengt und den verbleibenden Rückstand gegebenenfalls nach üblichen Methoden, wie Umkristallisation oder Chromatographie, von eventuell vorhandenen Verunreinigungen befreit.

Die bei der Durchführung der zweiten Stufe des erfindungsgemäßen Verfahrens als Ausgangssubstanzen benötigten Verbindungen der Formel (Ia) sind erfindungsgemäße Stoffe.

Die bei der Durchführung des erfindungsgemäßen Verfahrens in der zweiten Stufe als Reaktionskomponenten benötigten Halogen-Verbindungen sind durch die Formel (III) allgemein definiert.
- R⁴: steht bevorzugt für Methyl oder Ethyl.
- Hal: steht bevorzugt für Brom oder Iod.

Die Halogen-Verbindungen der Formel (III) sind bekannt.

Als Säurebindemittel kommen bei der Durchführung der zweiten Stufe des erfindungsgemäßen Verfahrens alle üblichen anorganischen oder organischen Basen in Frage. Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der zweiten Stufe des erfindungsgemäßen Verfahrens alle für derartige Umsetzungen üblichen, inerten organischen Solventien in Betracht. Vorzugsweise verwendbar sind Ether, wie Diethylether, Methyl-tert.-butyl-ether, Ethylenglykol-dimethylether, Tetrahydrofuran und Dioxan, ferner Nitrile, wie Acetonitril, und außerdem stark polare Solventien, wie Dimethylsulfoxid oder Dimethylformamid.

Die Reaktionstemperaturen können bei der Durchführung der zweiten Stufe des erfindungsgemäßen Verfahrens innerhalb eines größeren Bereiches variiert werden. Im allgemeinen arbeitet man bei Temperaturen zwischen 0°C und 120°C. vorzugsweise zwischen 20°C und 100°C.

Bei der Durchführung der zweiten Stufe des erfindungsgemäßen Verfahrens setzt man auf 1 Mol an Mercapto-imidazolyl-Derivat der Formel (Ia) im allgemeinen 1 bis 2 Mol an Halogen-Verbindung der Formel (III) sowie eine äquivalente Menge oder auch einen Überschuß an Säurebindemittel ein. Die Aufarbeitung erfolgt nach üblichen Methoden. Im allgemeinen geht man so vor, daß man das Reaktionsgemisch mit wäßriger Base und einem mit Wasser wenig mischbaren organischen Lösungsmittel versetzt, die organische Phase abtrennt, trocknet und einengt. Das erhaltene Produkt kann gegebenenfalls nach üblichen Methoden, z.B. durch Umkristallisation, von noch vorhandenen Verunreinigungen befreit werden.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes. Basidiomycetes bzw. Deuteromycetes.

Bakterizide Mittel werden im Pflanzenschutz eingesetzt zur Bekämpfung von Pseudomonadaceae, Rhizobiaceae, Enterobacteriaceae, Corynebacteriaceae bzw. Streptomycetaceae.

Beispielhaft aber nicht begrenzend seien einige Erreger von pilzlichen und bakteriellen Erkrankungen, die unter die oben aufgezählten Oberbegriffe fallen. genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz. Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Scterophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden. wie Lösungen, Emulsionen, Suspensionen, Pulver, Schäume, Pasten, Granulate. Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen. gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline. chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon. Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint. welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle. wie Kaoline. Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor. Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung im Pflanzenschutz als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen beispielsweise die folgenden Stoffe infrage.

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid;(E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Didobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat. Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol. Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin. Probenazol, Prochloraz, Procymidon, Propamocarb, Propiconazole, Propineb. Pyrazophos, Pyrifenox, Pyrimethanil, Pyroquilon,
Quintozen (PCNB),
Schwefel und Schwefel-Zubereitungen,
Tebuconazol, Tecloftalam, Technazen, Tetraconazol, Thiabendazol, Thicyofen. Thiophanat-methyl, Thiram, Tolclophos-methyl, Tolylfluanid, Triadimefon. Triadimenol, Triazoxid, Trichlamid, Tricyclazol, Tridemorph, Triflumizol, Triforin. Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A. Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Didiphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb. Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb. Isoxathion, Ivermectin, Lambda-cyhalothrin, Lufenuron,
Malathion, Mecarbam, Mevinphos, Mesulfenphos, Metaldehyd. Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

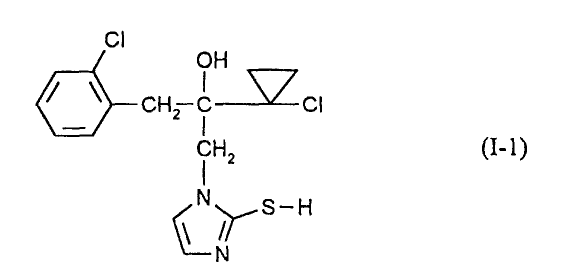

Ein Gemisch aus 1,55 g (5 mmol) 2-(1-Chlor-cyclopropyl)-1-(2-chlor-phenyl)-3-(imidazol-1-yl)-propan-2-ol, 0,32 g (0,01 g-Atom) Schwefel-Pulver und 20 ml absolutem N-Methyl-pyrrolidon wird unter Rühren 3 Stunden auf 200°C erhitzt. Anschließend wird das Rekationsgemisch mit Essigsäureethylester verdünnt und mehrfach mit gesättigter, wäßriger Ammoniumchlorid-Lösung ausgeschüttelt. Danach wird die organische Phase über Natriumsulfat getrocknet und unter vermindertem Druck eingeengt. Das verbleibende Produkt wird mit Petrolether/Essigsäureethylester = 1:1 als Laufmittel an Kieselgel chromatographiert. Nach dem Einengen des Eluates erhält man 0,6 g (35 % der Theorie) an 2-(1-Chlor-cyclopropyl)-1-(2-chlor-phenyl)-3-(2-mercapto-imidazol-1-yl)-propan-2-ol in Form eines Feststoffes vom Schmelzpunkt 124 bis 126°C.
¹H-NMR-Spektrum (200 MHz; CDCl₃; TMS): δ = 0,6 - 1,1 (m,4H); 3,0 (d,1H); 3,75 (d,1H); 4,4 (AB,2H); 5,15 (OH); 6,7 (1H); 7,05 (1H); 7,2 - 7,6 (m,4H); 10,25 (1H) ppm

### Herstellung der Ausgangssubstanz

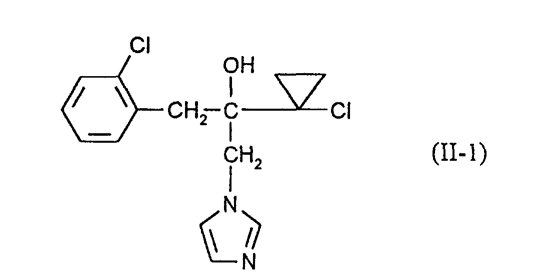

Ein Gemisch aus 5,56 g (20 mmol) 3-Chlor-2-(1-chlor-cyclopropyl)-1-(2-chlorphenyl)-propan-2-ol, 1,36 g (20 mmol) Imidazol und 4,15 g (30 mmol) wasserfreies Kaliumcarbonat in 30 ml absolutem Dimethylformamid wird 23 Stunden unter Rühren auf 100°C erhitzt. Anschließend wird das Reaktionsgemisch mit Essigsäureethylester verdünnt und mehrfach mit gesättigter, wäßriger Ammoniumchlorid-Lösung extrahiert. Danach wird die organische Phase über Natriumsulfat getrocknet und unter vermindertem Druck eingeengt. Das verbleibende Produkt wird mit Essigsäureethylester als Laufmittel an Kieselgel chromatographiert. Nach dem Einengen des Eluates unter vermindertem Druck erhält man 1.7 g (27 % der Theorie) an 2-(1-Chlor-cyclopropyl)-1-(2-chlor-phenyl)-3-(imidazol- 1 -yl)-propan-2-ol in Form einer Festsubstanz vom Schmelzpunkt 136 bis 139°C.
¹H-NMR-Spektrum (400 MHz; CDCl₃; TMS): δ = 0,5 - 0,8 (m,4H); 3,3 (d,1H); 3,45 (d,1H); 4,0 (d,1H); 4,6 (d,1H); 7,0 (m,2H); 7,25 - 7,5 (m,4H): 7,55 (s,1H) ppm.

### Beispiel A

| Erysiphe-Test (Gerste) / protektiv | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperqtur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

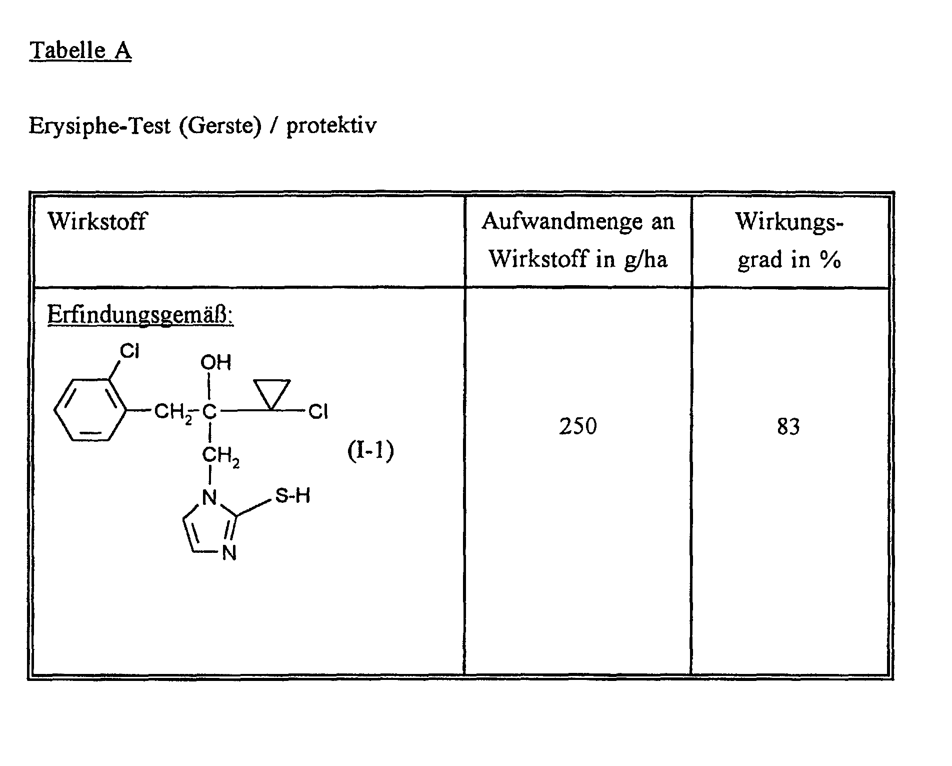

### Beispiel B

| Spaherotheca-Test (Gurke) / protektiv | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Sphaerotheca fuliginea inokuliert. Die Pflanzen werden bei ca. 23°C und einer relativen Luftfeuchtigkeit von ca. 70 % im Gewächshaus aufgestellt.

10 Tage nach der Inokulation erfolgt die Auswertung. Dabei bedeutet 0 % ein Wirkungsgrad, der demjenigen der Kontrolle entspricht, während 100 % bedeutet. daß kein Befall beobachtet wird.

Wirkstoffe, Wirkstoffkonzentrationen und Versuchsergebnisse gehen aus der folgenden Tabelle hervor.

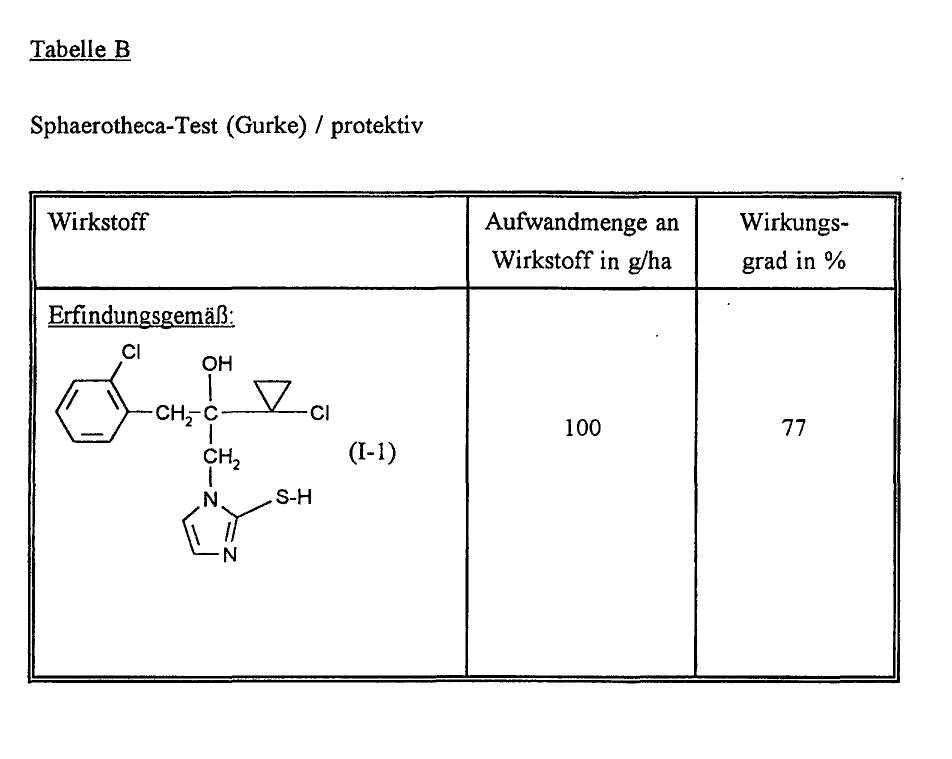

### Beispiel C

| Venturia-Test (Apfel) / protektiv | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während 100 % bedeutet, daß kein Befall beobachtet wird.

Wirkstoffe, Wirkstoffkonzentrationen und Versuchsergebnisse gehen aus der folgenden Tabelle hervor.

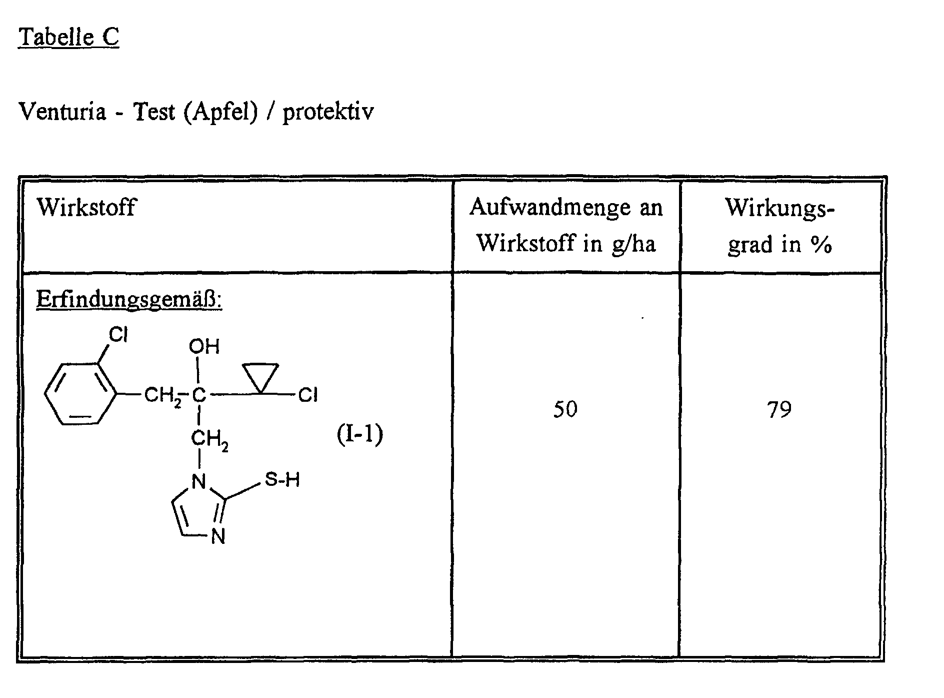

## Patentansprüche

1. Mercapto-imidazol-Derivate der Formel 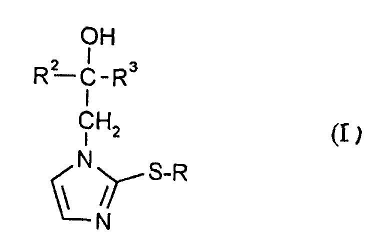 in welcher
R für Wasserstoff, Methyl, Ethyl, n - Propyl oder Isopropyl steht,
R² für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy. Methylthio, Trifluormethyl, Trifluormethoxy, Trifluormethylthio. Chlordifluormethoxy, Difluormethoxy, Chlordifluormethylthio. Methoxycarbonyl, Ethoxycarbonyl, Methoximinomethyl, 1-Methoximinoethyl, Nitro und/oder Cyano,
und
R³ für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl.

2. Mercapto - imidazol - Derivat gemäß Anspruch 1, **gekennzeichnet durch** die Formel

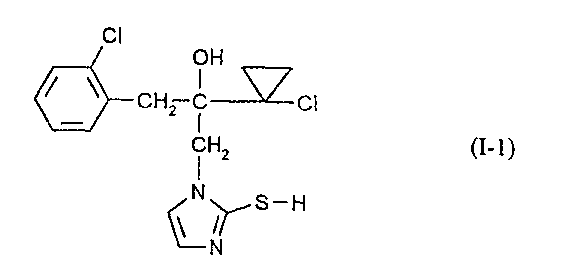

3. Verfahren zur Herstellung von Mercapto-imidazolyl-Derivaten der Formel (I) gemäß Anspruch 1,
**dadurch gekennzeichnet, daß** man Imidazole der Formel 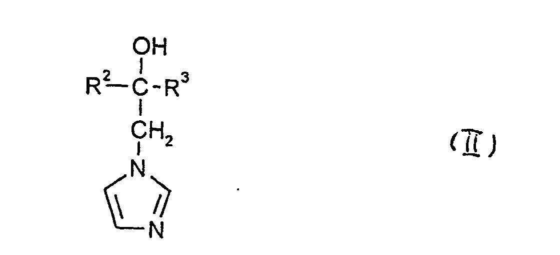 in welcher
R² und R ³ die oben angegebenen Bedeutungen haben,
entweder
a) nacheinander mit starken Basen und Schwefel in Gegenwart eines Verdünnungsmittels umsetzt und dann mit Wasser, gegebenenfalls in Gegenwart einer Säure hydrolysiert, oder
b) mit Schwefel in Gegenwart eines hoch siedenden Verdünnungsmittels umsetzt und dann gegebenenfalls mit Wasser sowie gegebenenfalls mit Säure behandelt,
und gegebenenfalls die nach den Varianten (a) und (b) entstehenden Verbindungen der Formel 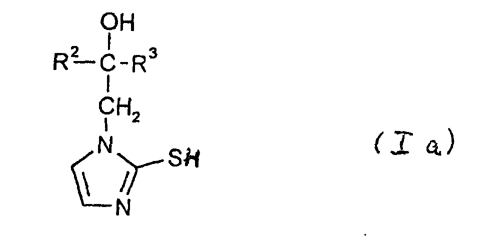 in welcher
R² und R ³ die oben angegebenen Bedeutungen haben,
mit Halogen-Verbindungen der Formel
R⁴-Hal (III)
in welcher
R⁴ für Methyl, Ethyl, n- Propyl oder Isopropyl steht und
Hal für Chlor, Brom oder Iod steht,
in Gegenwart eines Säurebindemittels und in Gegenwart eines Verdünnungsmittels umsetzt.

4. Mikrobizide Mittel, **gekennzeichnet durch** einen Gehalt an mindestens einem Mercapto-imidazolyl-Derivat der Formel (I) gemäß Ansprach 1 neben Streckmitteln und/oder oberflächenaktiven Stoffen.

5. Verwendung von Mercapto-imidazolyl-Derivaten der Formel (I) gemäß Anspruch 1 als Mikrobizide im Pflanzenschutz und im Materialschutz.

6. Verfahren zur Bekämpfung von unerwünschten Mikroorganismen im Pflanzenschutz und im Materialschutz, **dadurch gekennzeichnet, daß** man Mercapto-imidazolyl-Derivate der Formel (I) gemäß Anspruch 1 auf die Mikroorganismen und/oder deren Lebensraum ausbringt.

7. Verfahren zur Herstellung von mikrobiziden Mitteln, **dadurch gekennzeichnet, daß** man Mercapto-imidazolyl-Derivate der Formel (I) gemäß Anspruch 1 mit Streckmitteln und/oder oberflächenaktiven Stoffen vermischt.

## Claims

1. Mercapto-imidazolyl derivatives of the formula 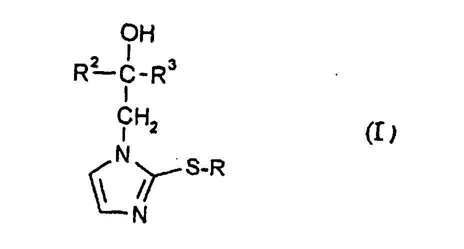 in which
R represents hydrogen, methyl, ethyl, n-propyl or isopropyl,
R² represents phenylalkyl having 1 to 4 carbon atoms in the straight-chain or branched alkyl moiety, it being possible for the phenyl moiety to be monosubstituted or trisubstituted by identical or different substituents from the series consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoxyiminomethyl, 1-methoxyiminoethyl, nitro and/or cyano,
and
R³ represents cycloalkyl having 3 to 6 carbon atoms, it being possible for each of these radicals to be monosubstituted to trisubstituted by identical or different substituents from the series consisting of fluorine, chlorine, bromine, cyano, methyl, ethyl, propyl, isopropyl and/or tert-butyl.

2. Mercapto-imidazolyl derivatives according to Claim 1, **characterized by** the formula

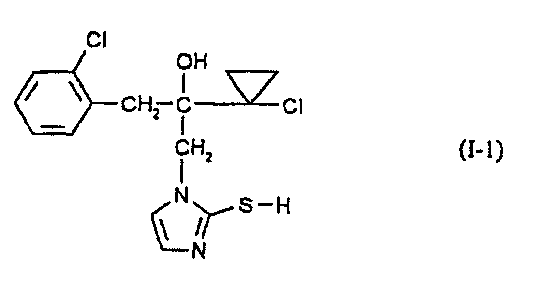

3. Process for the preparation of mercapto-imidazolyl derivatives of the formula (I) according to Claim 1, **characterized in that** imidazoles of the formula 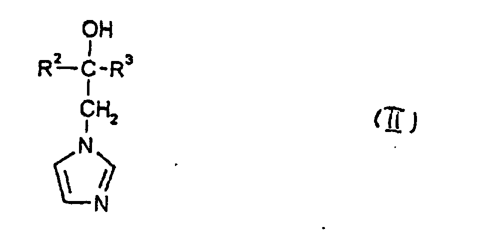 in which
R² and R³ have the abovementioned meanings,
are either
a) reacted in succession with strong bases and sulphur in the presence of a diluent and then hydrolysed with water, if appropriate in the presence of an acid, or
b) reacted with sulphur in the presence of a high-boiling diluent and then, if appropriate, treated with water and, if appropriate, with acid,
and, if appropriate, the compounds of the formula 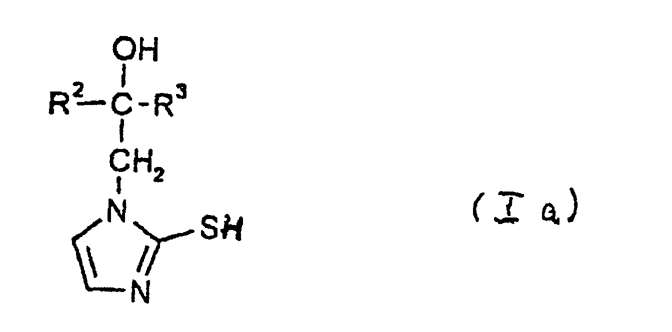 in which
R² and R³ have the abovementioned meanings
which result from variants (a) and (b)
are reacted with halogen compounds of the formula
R⁴-Hal (III)
in which
R⁴ represents methyl, ethyl, n-propyl or isopropyl and
Hal represents chlorine, bromine or iodine,
in the presence of an acid binder and in the presence of a diluent.

4. Microbicidal composition, **characterized by** a content of at least one mercapto-imidazolyl derivative of the formula (I) according to Claim 1, in addition to extenders and/or surface-active agents.

5. Use of mercapto-imidazolyl derivatives of the formula (I) according to Claim 1 as microbicides in crop protection and in the protection of materials.

6. Method for controlling undesired microorganisms in crop protection and in the protection of materials, **characterized in that** mercapto-imidazolyl derivatives of the formula (I) according to Claim 1 are applied to the microorganisms and/or their environment.

7. Process for the preparation of microbicidal compositions, **characterized in that** mercapto-imidazolyl derivatives of the formula (I) according to Claim 1 are mixed with extenders and/or surface-active substances.

## Revendications

1. Dérivés mercapto-imidazoliques de formule 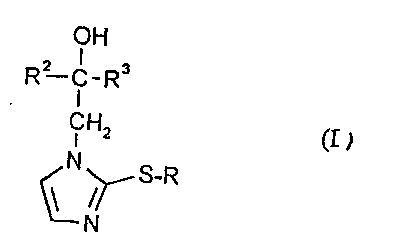 dans laquelle
R est l'hydrogène, un reste méthyle, éthyle, n-propyle ou isopropyle,
R² est un reste phénylalkyle ayant 1 à 4 atomes de carbone dans la partie alkyle linéaire ou ramifiée, la partie phényle pouvant porter 1 à 3 substituants, identiques ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarboxyle, éthoxycarbonyle, méthoxyiminométhyle, 1-méthoxy-iminoéthyle, nitro et/ou cyano,
et
R³ est un reste cycloalkyle ayant 3 à 6 atomes de carbone, chacun des restes ainsi définis pouvant porter 1 à 3 substituants, identiques ou différents, fluoro, chloro, bromo, cyano, méthyle, éthyle, propyle, isopropyle et/ou tertiobutyle.

2. Dérivé mercapto-imidazolique suivant la revendication 1, **caractérisé par** la formule :

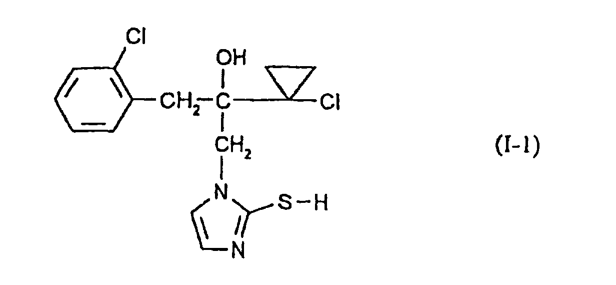

3. Procédé de production de dérivés de mercapto-imidazolyle de formule (I) suivant la revendication 1,
**caractérisé en ce que** des imidazoles de formule 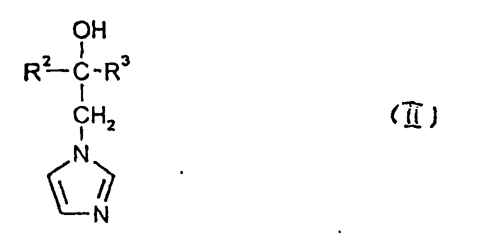 dans laquelle
R² et R³ ont les définitions indiquées ci-dessus,
ou bien
a) sont amenés à réagir successivement avec des bases fortes et du soufre en présence d'un diluant puis hydrolysés par l'eau, le cas échéant en présence d'un acide,
ou bien
b) sont amenés à réagir avec du soufre en présence d'un diluant de haut point d'ébullition puis traités éventuellement avec l'eau, ainsi que le cas échéant avec un acide,
et le cas échéant les composés produits selon les variantes (a) et (b) de formule 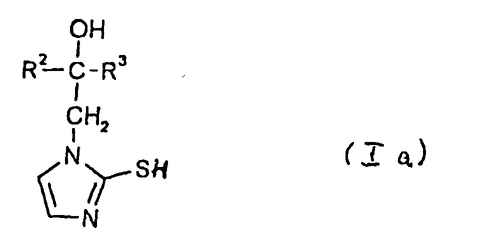
dans laquelle
R² et R³ ont les définitions indiquées ci-dessus sont amenés à réagir avec des composés halogénés de formule
R⁴-Hal (III)
dans laquelle
R⁴ est un reste méthyle, éthyle, n-propyle ou isopropyle et
Hal représente le chlore, le brome ou l'iode,
en présence d'un accepteur d'acide et en présence d'un diluant.

4. Compositions microbicides, **caractérisées par** une teneur en au moins un dérivé de mercapto-imidazolyle de formule (I) suivant la revendication 1, à côté de diluants et/ou d'agents tensioactifs.

5. Utilisation de dérivés de mercapto-imidazolyle de formule (I) suivant la revendication 1 comme microbicides dans la protection des plantes et dans la protection des matériaux.

6. Procédé pour combattre des micro-organismes indésirables dans la protection des plantes et dans la protection des matériaux, **caractérisé en ce qu'**on épand des dérivés de mercapto-imidazolyle de formule (I) suivant la revendication 1 sur les micro-organismes et/ou sur leur milieu.

7. Procédé de préparation de compositions microbicides, **caractérisé en ce qu'**on mélange des dérivés de mercapto-imidazolyle de formule (I) suivant la revendication 1 avec des diluants et/ou des agents tensioactifs.
